# EUROPÄISCHE PATENTANMELDUNG

(11) **EP 4 725 308 A1**
(43) Veröffentlichungstag der Anmeldung: **15.04.2026**
(21) Anmeldenummer: 24206203.2
(22) Anmeldetag: 11.10.2024
(51) Int. Cl.: A01M 1/02, A01G 9/02, A01K 67/02, A01K 67/30

(54) **ANSIEDLUNGSVORRICHTUNG**

(71) Anmelder: LGU GmbH, 34613 Schwalmstadt (DE)
(72) Erfinder: HAHN, Florian, 34613 Schwalmstadt Treysa (DE)
(74) Vertreter: Habermann Intellectual Property Partnerschaft von Patentanwälten mbB

(57) **Zusammenfassung**

Eine Ansiedlungsvorrichtung (1) zur Unterstützung der Ansiedlung von mittleren bis kleinen Lebewesen bis hin zu Mikroorganismen, Pflanzen und Pilzen weist einen Innenraum auf, der von einem auf einem Untergrund aufstellbaren Boden (3) und einem gegenüberliegenden Dach (4) sowie von vier sich jeweils von dem Boden (3) bis zu dem Dach (4) erstreckenden Seitenwänden umgeben ist. Zwei einander gegenüberliegende Seitenwände bilden Abschirmungswände (5), die durch Wandelemente (6) verschlossen sind. Zwei weitere einander gegenüberliegende und auf beiden Seiten an eine Abschirmungswand (5) angrenzende Seitenwände bilden Öffnungswände (7), die jeweils eine Anzahl von Öffnungen (8) aufweisen, durch welche hindurch der Innenraum zugänglich ist. In jeder Öffnungswand (7) ist hinter mehreren Öffnungen (8) jeweils ein Ansiedlungsgang (9) ausgebildet und durch die zugeordnete Öffnung (8) zugänglich, der sich von der betreffenden Öffnung (8) in den Innenraum hinein erstreckt und an einem der Öffnung (8) entgegenliegenden Ende des Ansiedlungsgangs (9) verschlossen sein kann. Mehrere Ansiedlungsgänge (9) weisen jeweils einen Gangboden (10) mit einem sich von der Öffnung (8) in den Innenraum hinein zu dem Boden (3) hin gerichteten Ganggefälle zwischen 5° und 33°, vorzugsweise zwischen 20° und 30° relativ zu dem Boden (3) der Ansiedlungsvorrichtung (1) auf.

## Beschreibung

Die Erfindung betrifft eine Ansiedlungsvorrichtung zur Unterstützung der Ansiedlung von kleinen Lebewesen, mit einem Innenraum, der von mindestens zwei Seitenwänden umgeben ist, die sich von einem auf einem Untergrund aufstellbaren Boden bis zu einer dem Boden gegenüberliegenden Oberseite der Ansiedlungsvorrichtung erstrecken, wobei mindestens eine Seitenwand eine Abschirmungswand bildet, die durch Wandelemente verschlossen ist, und mindestens eine an die Abschirmungswand angrenzende Seitenwand eine Öffnungswand bildet, die jeweils eine Anzahl von Öffnungen aufweist, durch welche hindurch der Innenraum zugänglich ist.

Durch den Einfluss der Menschen auf die Umwelt verändern sich die Lebensbedingungen von Pflanzen und Tieren. Durch großflächige Strukturen wie beispielsweise bei bewirtschafteten Ackerflächen, die zeitweilig oder dauerhaft für Monokulturen genutzt werden, geht die Artenvielfalt in diesen Naturbereichen zurück, da sich die Lebensbedingungen für viele Tiere und Pflanzen grundlegend verändern und oftmals ein zum Überleben ausreichendes Angebot an Lebensraum und Nahrung knapp ist oder fehlt.

Es ist beispielsweise aus dem städtischen Raum bekannt, Ansiedlungsvorrichtungen in Gärten oder an Wohnanlagen zu errichten, mit denen Tieren oder Pflanzen möglichst günstige Lebensbedingungen geboten werden sollen. So werden beispielsweise insbesondere in der kalten Jahreszeit Vogelhäuser aufgestellt oder aufgehängt und mit Vogelfutter befüllt. Mit Nistkästen werden Nistmöglichkeiten für Vögel angeboten. Brachliegende Garten- oder Parkflächen oder von Menschen absichtlich naturnah belassene Grünflächen ziehen viele Insekten, Vögel und kleine Säugetiere sowie Pflanzen und Mikroorganismen an, die dort einen Lebensraum und ausreichend Nahrung finden können. Es sind weiterhin Unterschlupf- und Nisthilfen für Insekten bekannt, bei denen mehrere Kammern oder Fächer mit unterschiedlichen üblicherweise natürlichen Materialien befüllt sind, in denen sich Insekten wohlfühlen.

Bei der Nutzung von Naturflächen außerhalb eines städtischen Raums wird zunehmend Wert darauf gelegt, dass trotz einer Bewirtschaftung der Naturflächen beispielsweise Randbereiche möglichst nicht bewirtschaftet werden oder so bepflanzt und gepflegt werden, dass für Tiere vorteilhafte Lebensbedingungen begünstigt werden. Oftmals werden auch für Tiere oder für eine Artenvielfalt von Tieren und Pflanzen möglichst vorteilhafte Rahmenbedingungen für die Bewirtschaftung der Naturflächen vorgegeben.

Es sind auch vereinzelt Ansiedlungsvorrichtungen aus der Praxis bekannt geworden, die künstlich hergestellt und in einem städtischen oder natürlichen Umfeld aufgestellt oder errichtet werden, mit denen gezielt die Ansiedlung von verschiedenen Tier- oder Pflanzenarten unterstützt und gefördert werden soll. Solche Ansiedlungsvorrichtungen dienen dazu, die Individuenanzahl von Tieren oder Pflanzen zu erhöhen, die sich langfristig an oder in einer solchen Ansiedlungsvorrichtung aufhalten oder dort ansiedeln, und sollen die genetische Vielfalt und die Biodiversität steigern.

Es hat sich gezeigt, dass eine Ansiedlungsvorrichtung möglichst vielen und unterschiedlichen kleinen Lebewesen einen vorteilhaft nutzbaren Lebensraum bieten sollte, da durch die Ansiedlung verschiedener kleiner Lebewesen nicht nur die Biodiversität gefördert wird, sondern auch für die verschiedenen Lebewesen die Lebensbedingungen verbessert und ein möglichst naturnahes Ökosystem zur Verfügung gestellt werden kann. Als kleine Lebewesen werden insbesondere Insekten, Vögel und kleinen Säugetiere, aber auch Pflanzen und Pilze sowie beispielsweise Bakterien, Archaeen und Eukaryoten angesehen.

Es wird deshalb als eine Aufgabe der vorliegenden Erfindung angesehen, eine Ansiedlungsvorrichtung der eingangs genannten Gattung so auszugestalten, dass mit einem möglichst kleinen Flächenbedarf möglichst effizient für verschiedene Tierarten und Pflanzenarten möglichst vorteilhafte Lebensbedingungen geboten werden, sodass sich möglichst viele Tiere und Pflanzen an oder in einer solchen Ansiedlungsvorrichtung ansiedeln.

Diese Aufgabe wird erfindungsgemäß dadurch gelöst, dass in der Öffnungswand hinter mehreren Öffnungen jeweils ein Ansiedlungsgang ausgebildet und durch die zugeordnete Öffnung zugänglich ist, der sich von der betreffenden Öffnung in den Innenraum hinein erstreckt, und dass jeder Ansiedlungsgang durch Gangwände von anderen Ansiedlungsgängen getrennt ist. Die einzelnen Ansiedlungsgänge bieten kleinen Lebewesen einen günstigen Lebensraum. In den Ansiedlungsgängen können sich die Lebewesen zurückziehen oder dort ansiedeln und sind geschützt vor unwirtlichen Umgebungsbedingungen. Mehrere Öffnungen weisen zweckmäßigerweise eine ausreichend große Querschnittsfläche auf, die beispielsweise für kleine Nagetiere oder Insektenfresser einen Zugang in den dahinter ausgebildeten Ansiedlungsgang ermöglicht. Zu diesem Zweck weist die Querschnittsfläche der Öffnungen beispielsweise eine Fläche von mehr als 25 cm2 oder mehr als 50 cm2 auf. Die Querschnittsfläche solcher Öffnungen kann auch einen größten Durchmesser von mehr als 5 cm oder von mehr als 10 cm aufweisen, wobei ein kleinster Durchmesser nicht kleiner als 3 cm oder 2 cm sein sollte. Ein Ansiedlungsgang kann sich ausgehend von einer Öffnung in einer Öffnungswand durch die gesamte Ansiedlungsvorrichtung bis zu einer gegenüberliegenden Öffnungswand oder zu einem beabstandeten Bereich derselben Öffnungswand hin erstrecken und auch dort über eine zugeordnete Öffnung zugänglich sein. Die beiden Öffnungen können unterschiedliche Größen bzw. Querschnittsflächen aufweisen. In vorteilhafter Weise kann vorgesehen sein, dass der Ansiedlungsgang keinen gradlinigen Verlauf aufweist, wobei von keiner Öffnung aus die jeweils gegenüberliegende Öffnung sichtbar ist bzw. durch eine innerhalb des Ansiedlungsgangs verlaufende Gerade verbunden werden kann.

Gemäß einer ebenfalls vorteilhaften Ausgestaltung des Erfindungsgedankens kann vorgesehen sein, dass mehrere oder alle Ansiedlungsgänge jeweils in einem Abstand von der zugeordneten Öffnung verschlossen sind. Durch das verschlossene Ende der Ansiedlungsgänge wird verhindert, dass ein für viele Tiere und Pflanzen unangenehmer Luftzug bzw. Durchzug durch einen solchen Ansiedlungsgang entstehen kann.

Zudem können sich Bodenmaterialien wie beispielsweise Erde oder Sand sowie Laub oder Pflanzenreste in den Ansiedlungsgängen ansammeln oder von den Tieren dort angesammelt werden. Die an einem Ende verschlossenen Ansiedlungsgänge können von den Tieren auch als Lager für gesammelte Nahrung und als Nest bzw. als Schlafstätte genutzt werden.

Einer als besonders vorteilhaft erachteten Ausgestaltung des Erfindungsgedankens zufolge ist vorgesehen, dass mehrere Ansiedlungsgänge, die von einer Öffnungswand aus zugänglich sind, jeweils einen Gangboden mit einem sich von der Öffnung in den Innenraum hinein zu dem Boden hin gerichteten Ganggefälle zwischen 5° und 33°, vorzugsweise zwischen 20° und 30° relativ zu dem Boden der Ansiedlungsvorrichtung aufweisen. Es hat sich gezeigt, dass die zur Mitte hin schräg nach unten verlaufenden Ansiedlungsgänge insbesondere von krabbelnden Insekten und kleinen Säugetieren aufgesucht und intensiv genutzt werden. Durch den schräg nach unten gerichteten Verlauf kann jeder Ansiedlungsgang für viele verschiedene Tierarten behaglich und für eine Ansiedlung einladend ausgebildet und ausgestaltet sein.

So hat sich gezeigt, dass beispielsweise lockerer Sand bis zu einem natürlichen Böschungswinkel von etwa 33° eine Hangfläche ausbilden kann, sodass ein Gangboden eines Ansiedlungsgangs auch mit lockerem Sand weitgehend oder vollständig bedeckt sein kann. Durch das Ganggefälle wird zudem erreicht, dass ein in den Ansiedlungsgang eingebrachtes oder sich dort ansammelndes Gangbodenmaterial dauerhaft in dem Ansiedlungsgang verbleibt und das Gangbodenmaterial, beispielsweise Sand oder Erdreich, nicht durch übliche Witterungseinflüsse wie Wind oder Regen weitgehend oder vollständig aus dem Ansiedlungsgang herausgeblasen oder herausgeschwemmt werden kann. Falls sich Erde, Laubreste oder Stöckchen von Pflanzen auf natürliche Weise in einem Ansiedlungsgang ansammeln oder durch die Pflege von Menschen oder durch das Ansammeln von Tieren dort absichtlich gelagert werden, kann sich eine dauerhafte Bedeckung des schräg geneigt verlaufenden Gangbodens ausbilden, die auch beispielsweise durch äußere Einflüsse wie Wind oder Regen nicht ohne weiteres gelöst und nahezu vollständig in einen tiefsten Bereich des Ansiedlungsgangs geblasen oder geschwemmt wird. Durch den schräg nach unten verlaufenden Ansiedlungsgang wird unterhalb einer Unterkante der zugeordneten Öffnung in der Öffnungswand ein Hohlraum bereitgestellt, der für viele Tiere einen attraktiven Rückzugs- und Nestbereich bildet. Zudem wird durch den schräg nach unten verlaufenden und an dessen Ende verschlossenen Ansiedlungsgang erreicht, dass durch das Gefälle mit der Zeit jegliches Substrat, welches sich in einem Ansiedlungsgang ansammelt, von der Öffnung weg in Richtung des verschlossenen Endes wandert und sich dort ansammelt.

In einer Öffnungswand können wenige Öffnungen über eine Wandfläche der Öffnungswand verteilt und beabstandet zueinander angeordnet sein, die jeweils in einen Ansiedlungsgang führen, dessen Querschnittsfläche größer als eine Querschnittsfläche der Öffnung sein kann. Um möglichst viele Ansiedlungsgänge in einem vorgegebenen Raumvolumen des Innenraums der Ansiedlungsvorrichtung ausbilden und bereitstellen zu können kann gemäß einer vorteilhaften Ausgestaltung des Erfindungsgedankens vorgesehen sein, dass jede Öffnungswand durch aneinander angrenzende Öffnungen gebildet wird, die jeweils durch Gangwände begrenzt sind, welche die sich von den Öffnungen in den Innenraum erstreckenden Ansiedlungsgänge begrenzen. Zumindest innerhalb eines Randbereichs, der an die Wandfläche der Öffnungswand angrenzt, weist die Ansiedlungsvorrichtung vorzugsweise sich quer zur Öffnungswand erstreckende Gangwandabschnitte auf, die sich von weiter Innen bis zu der zugeordneten Öffnung hin erstrecken, einen Öffnungsrand der Öffnung bilden und die Öffnung begrenzen. Auf diese Weise wird für die Ansiedlung von Tieren und Pflanzen ein möglichst großes Raumvolumen in den Ansiedlungsgängen bereitgestellt und der Innenraum zumindest in dem an die Wandfläche der Öffnungswand angrenzenden Randbereich lediglich durch gegebenenfalls dünn ausgebildete Gangwände durchzogen. Die Wandfläche der Öffnungswand wird dabei im Wesentlichen durch die Öffnungen gebildet, die lediglich entlang der jeweiligen Öffnungsränder durch quer zu der Wandfläche verlaufende und dort endende Gangwandabschnitte voneinander abgegrenzt sind.

Die Ansiedlungsvorrichtung kann ein rahmenartig ausgebildetes Tragwerk aufweisen, über welches die auf die Ansiedlungsvorrichtung üblicherweise wirkenden Kräfte und mechanischen Belastungen aufgenommen und auf einen Untergrund abgetragen werden können. In das rahmenartig ausgebildete Tragwerk können beispielsweise die Seitenwände und der Boden sowie eine Anzahl von sich in den dadurch begrenzten Innenraum erstreckenden Ansiedlungsgängen eingesetzt und daran festgelegt werden. Die in das Tragwerk eingesetzten oder daran festgelegten Komponenten wie beispielsweise die Ansiedlungsgänge können aus einem textilen Material oder aus einem Geflecht aus natürlichen Fasern oder für die Natur unbedenklichen Kunstfasern hergestellt sein. Es ist ebenfalls denkbar, dass die einzelnen Komponenten aus einem anderen Material bestehen oder eine Formgebung aufweisen, die nicht dazu geeignet ist, im Vergleich zu dem Tragwerk nennenswerte Kräfte und mechanische Belastungen aufnehmen zu können.

Gemäß einer besonders vorteilhaften Ausgestaltung des Erfindungsgedankens ist jedoch vorgesehen, dass die Gangwände der Ansiedlungsgänge zumindest bereichsweise plattenförmig ausgebildet sind und miteinander verbunden sind, um eine Stützstruktur der Ansiedlungsvorrichtung zu bilden, auf welcher bei einer bestimmungsgemäßen Nutzung der Ansiedlungsvorrichtung eine weitere Ansiedlungsvorrichtung abgestützt und getragen werden kann. Es hat sich gezeigt, dass mit geeignet ausgestalteten und angeordneten plattenförmigen Gangwänden nicht nur die einzelnen Ansiedlungsgänge vorgegeben und begrenzt werden können, sondern gleichzeitig eine formstabile und mechanisch belastbare Stützstruktur ausgebildet werden kann, sodass kein zusätzliches Tragwerk erforderlich ist. Dadurch können der für das Tragwerk erforderlich Raumbedarf eingespart und für Ansiedlungsgänge oder andere Bereiche oder Elemente verwendet werden, die eine Ansiedlung von Tieren und Pflanzen begünstigen.

Die Ansiedlungsvorrichtung kann auch weitgehend oder vollständig aus plattenförmigen Gangwänden und damit aus einem einheitlichen Material hergestellt werden, was nach der bestimmungsgemäßen Nutzung der Ansiedlungsvorrichtung eine umweltgerechte Wiederaufbereitung des Materials der Gangwände erleichtert und eine aufwändige Materialtrennung unnötig werden lässt. Die Gangwände können aus geeignet zugeschnittenen ebenflächigen Materialplatten hergestellt werden, die anschließend zu den Ansiedlungsgängen und weiterhin zu der Ansiedlungsvorrichtung zusammengefügt werden können. Dabei können die einzelnen Materialplatten entweder über geeignet ausgebildete Verbindungsbereiche formschlüssig zusammengesteckt werden, oder aber mit geeigneten Hilfsmitteln wie beispielsweise umweltfreundlichen Klebemitteln und gesonderten Befestigungsmitteln miteinander verbunden werden.

Es ist ebenfalls denkbar, dass die Ansiedlungsvorrichtung mit Hilfe eines geeigneten additiven Fertigungsverfahrens wie beispielsweise Fused Deposition Modeling oder Selektives Laser-Sintern einstückig hergestellt wird. Die Verwendung eines derartigen additiven Fertigungsverfahrens ermöglicht es, Ansiedlungsvorrichtungen mit einer komplexen Formgebung der einzelnen Ansiedlungsgänge in einer wirtschaftlich sinnvollen Weise herzustellen. Mit geeigneten 3D-Druckvorrichtungen können verschiedene Materialien für additive Fertigungsverfahren verwendet werden, die umweltverträglich sind und sowohl während der bestimmungsgemäßen Nutzung der Ansiedlungsvorrichtung für Tiere und Pflanzen vorteilhafte Eigenschaften aufweisen als auch nach einer bestimmungsgemäßen Verwendung mit geringem Aufwand wieder aufbereitet oder umweltgerecht entsorgt werden können.

In vorteilhafter Weise ist vorgesehen, dass die Gangwände der Ansiedlungsgänge mindestens abschnittsweise porös ausgebildet sind oder eine Anzahl kleiner Klimaöffnungen aufweisen, durch welche ein Austausch von Luft und Feuchtigkeit zwischen benachbarten Ansiedlungsgängen ermöglicht wird, ohne dass durch diese Klimaöffnungen auch nennenswerte Mengen an biologischem Material und insbesondere von dem jeweiligen Gangbodenmaterial wie beispielsweise Sand oder Erdreich von dem einen Ansiedlungsgang in einen benachbarten Ansiedlungsgang gelangen können.

Eine Querschnittsfläche eines Ansiedlungsgangs kann eine nahezu beliebige Formgebung aufweisen. Die Querschnittsfläche des Ansiedlungsgangs kann identisch zu der Querschnittsfläche der zugeordneten Öffnung in der Öffnungswand sein. Die Querschnittsfläche des Ansiedlungsgangs kann über die gesamte Erstreckung des Ansiedlungsgangs im Wesentlichen gleichbleibend ausgebildet sein oder sich über den Verlauf des Ansiedlungsgangs hinweg verändern.

Optional ist vorgesehen, dass mindestens mehrere Ansiedlungsgänge über mindestens einen Abschnitt des jeweiligen Ansiedlungsgangs eine rautenförmige oder wabenförmige Querschnittsfläche aufweisen. Es hat sich gezeigt, dass Ansiedlungsgänge mit einer mindestens abschnittsweise rautenförmigen oder wabenförmigen Querschnittsfläche wegen der ebenen Bereiche und der diese Bereiche begrenzenden Kanten nicht nur für die Ansiedlung von Tieren und Pflanzen vorteilhafte räumliche Rahmenbedingungen schaffen, sondern auch mit geringem Materialaufwand für die Gangwände vergleichsweise stabile und mechanisch belastbare Stützstrukturen ausbilden können. Bei einem geringen Eigengewicht und einem geringen Raumbedarf für dünnwandige Gangwände lassen sich auf diese Weise formstabile Ansiedlungsvorrichtungen herstellen, die eine große Menge an natürlichem Material wie beispielsweise Sand und Erde, aber auch Pflanzen und Pflanzenreste sowie Feuchtigkeit und Wasser aufnehmen und in der Ansiedlungsvorrichtung anlagern kann, um einen möglichst ansprechenden Lebensraum für Insekten, Vögel und kleine Tiere sowie für Pflanzen und Mikroorganismen bieten zu können. Die einzelnen Ansiedlungsvorrichtungen können auch derart stabil und mechanisch belastbar ausgebildet sein, dass mehrere Ansiedlungsvorrichtungen übereinander oder in einem Verbund miteinander angeordnet und verwendet werden können, um in einem möglichst großen Raumvolumen das Ansiedeln von Tieren und Pflanzen begünstigen und fördern zu können.

Um Tieren und auch Pflanzen einen Wechsel von einem Ansiedlungsgang in einen anderen Ansiedlungsgang zu erleichtern kann optional vorgesehen sein, dass mehrere nebeneinander angeordnete Ansiedlungsgänge durch Gangwechselöffnungen miteinander verbunden sind. So können sich in einer Ansiedlungsvorrichtung größere Populationen einzelner Tierarten oder Pflanzenarten über mehrere Ansiedlungsgänge hinweg ausbreiten und einzelne Individuen durch die Gangwechselöffnungen hindurch über mehrere Ansiedlungsgänge bewegen und verbreiten, ohne dass dafür ein einzelner Ansiedlungsgang durch dessen Öffnung verlassen und ein weiterer Ansiedlungsgang nur durch dessen Öffnung in der Öffnungswand betreten oder erreicht werden kann.

Zudem kann auch natürliches Material, welches sich in den einzelnen Ansiedlungsgängen ansammelt oder absichtlich dort deponiert wurde, durch Gangwechselöffnungen hindurch in andere Ansiedlungsgänge entweder durch die Umgebungsbedingungen wie Wind oder Regen oder Schnee bzw. Eisbildung verlagert werden, oder durch Tiere oder Pflanzenwachstum durch Gangwechselöffnungen hindurch in andere Ansiedlungsgänge befördert werden. Derartiges Material kann auch zu einer Gesamtstabilität der Ansiedlungsvorrichtung beitragen. Die Gangwechselöffnungen sind zweckmäßigerweise so ausgebildet und angeordnet, dass zwischen manchen Ansiedlungsgängen ein Austausch von Regenwasser und Feuchtigkeit behindert zwischen anderen Ansiedlungsgängen ein solcher Austausch unterstützt und gefördert wird. Auf diese Weise können in einer Ansiedlungsvorrichtung mehrere unterschiedliche Umgebungsbedingungen für Tiere und Pflanzen erzeugt werden, die über längere Zeiträume hinweg entweder eher trocken und luftig oder aber eher feucht bzw. mit stehender Luft gehalten werden können. Die Gangwände können Gangwechselöffnungen aufweisen, die zwei aneinander angrenzende Ansiedlungsgänge miteinander verbinden und ein Überwechseln von kleinen Lebewesen oder Pflanzen aus einem Ansiedlungsgang in einen angrenzenden Ansiedlungsgang ermöglichen. Zwischen bei benachbart angeordneten, jedoch nicht unmittelbar aneinander angrenzend verlaufenden Ansiedlungsgängen können auch ein verbindender Gangwechselgang oder mehrere Gangwechselgänge ausgebildet sein, um eine möglichst hohe Konnektivität der Ansiedlungsgänge in dem Innenraum der Ansiedlungsvorrichtung zu ermöglichen.

Die Gangwechselöffnungen können auch strategisch so angelegt werden, dass ein Pflanzenwachstum über mehrere Ansiedlungsgänge hinweg begünstig wird und zudem zusätzlicher Raum und Halt für Wurzeln bereitgestellt wird. Die Öffnungsgröße derartiger Gangwechselöffnungen ist dabei zweckmäßigerweise so gewählt, dass ein radiales Dickenwachstum von Wurzeln nicht zu derart großen und starken Wurzeln führen kann, dass eine unbeabsichtigte Beschädigung oder Zerstörung der Ansiedlungsvorrichtung befürchtet werden muss.

Um für verschiedene Ansiedlungsgänge eine Nutzung durch unterschiedliche Tierarten zu begünstigen oder vorzugeben kann optional vorgesehen sein, dass die Öffnung eines Ansiedlungsgangs durch einen Zugangsbeschränkungsdeckel abgedeckt ist, der eine oder mehrere Zugangsöffnungen aufweist, durch welche hindurch ein Lebewesen sich in den Ansiedlungsgang hinein oder aus dem Ansiedlungsgang heraus bewegen kann. So kann ein Zugangsbeschränkungsdeckel eine größere Anzahl kleiner Zugangsöffnungen aufweisen, sodass nur kleine Insekten in der Lage sind, den dahinter befindlichen Ansiedlungsgang durch eine kleine Zugangsöffnung zu erreichen und zu nutzen. Andere und insbesondere in einem Abstand zu einem Untergrund angeordnete Ansiedlungsgänge können eine Öffnung mit einem Zugangsbeschränkungsdeckel aufweisen, der ein einziges großes Öffnungsloch wie bei einem Nistkasten für Vögel aufweist und gegebenenfalls in der Nähe des Öffnungslochs einen nach außen vorspringenden Stab oder Tritt aufweist, sodass Vögel besonders einfach durch die Zugangsöffnung in dem Zugangsbeschränkungsdeckel hindurch in den dahinter liegenden Ansiedlungsgang gelangen können und diesen wie einen Nistkasten als Nest für die Aufzucht Ihrer Jungvögel nutzen können. Es kann auch vorgesehen sein, dass ein Zugangsbeschränkungsdeckel eine Zugangsöffnung aufweist, die mit einem Gitter oder mit einem Textil bedeckt ist, sodass sich nur Tiere oder Pflanzen in dem betreffenden Ansiedlungsgang ansiedeln können, die durch das Gitter oder das Textil hindurch in den Ansiedlungsgang gelangen können.

Der Zugangsbeschränkungsdeckel kann einstückig mit der Öffnungswand ausgebildet sein und beispielsweise bei einer Herstellung der Ansiedlungsvorrichtung mit einem additiven Fertigungsverfahren während desselben Fertigungsschritt ausgebildet werden. Der Zugangsbeschränkungsdeckel kann auch gesondert hergestellt und nachträglich an einem Öffnungsrand einer Öffnung in der Öffnungswand festgelegt werden. Bei einer Verwendung von nachträglich an einer Öffnung festlegbaren Zugangsbeschränkungsdeckeln kann eine einheitlich ausgestaltete Öffnungswand einer Ansiedlungsvorrichtung individuell mit verschiedenen und jeweils unterschiedlich angeordneten Zugangsbeschränkungsdeckeln versehen werden, sodass eine einzelne Ansiedlungsvorrichtung entweder in Abhängigkeit des jeweils vorgesehenen Verwendungsorts oder aber in Abhängigkeit von Jahreszeiten oder wechselnden Umgebungsbedingungen durch unterschiedlich ausgestaltete Öffnungswände angepasst und vorgegeben werden kann.

In vorteilhafter Weise kann optional vorgesehen sein, dass mindestens eine Gangwand eines Ansiedlungsgangs von dem Innenraum heraus über eine Außenseite der Öffnungswand vorspringend ausgebildet ist, um eine Wandabschirmung zwischen Öffnungen auf gegenüberliegenden Seiten der vorspringenden Gangwand zu bilden. Mit einer derart vorspringend ausgebildeten Gangwand kann in einfacher Weise erreicht werden, dass rechts und links von dieser Wandabschirmung die dort angeordneten Öffnungen in der Öffnungswand voneinander abgetrennt werden und beispielsweise in den jeweiligen Ansiedlungsgängen nistende Vögel dort mehr Ruhe und Separierung erfahren können. Zudem wird ein Zugang zu benachbarten Ansiedlungsgängen dadurch erschwert, da kriechende oder kletternde Tiere nicht ohne weiteres zwischen Ansiedlungsgängen überwechseln können, die auf verschiedenen Seiten der Wandabschirmung angeordnet sind. Weiterhin können diese über die Öffnungswand herausgezogenen Gangwände eine Verschattung der benachbarten Öffnungen erzeugen. Je weiter eine solche Gangwand über die Öffnungswand vorspringend ausgebildet ist, umso höher ist die Verschattung sowie die Separierung. Durch die Verschattung kann eine direkte Sonneneinstrahlung in angrenzende Ansiedlungsgänge reduziert werden, was eine unerwünschte Verdunstung von Wasser und Feuchtigkeit aus dem Ansiedlungsgang heraus minimiert.

Gemäß einer Ausgestaltung des Erfindungsgedankens kann vorgesehen sein, dass in dem Innenraum ein mit Wasser befüllbares Wasserbecken ausgebildet ist. In einem solchen Wasserbecken kann ein Wasservorrat für die Lebewesen in der Ansiedlungsvorrichtung angesammelt und bereitgehalten werden. Das Wasserbecken kann beispielsweise säulenförmig oder plattenförmig ausgebildet sein und sich von einem Bereich nahe dem Boden bis zu der Oberseite der Ansiedlungsvorrichtung hin erstrecken, sodass viele Ansiedlungsgänge an das Wasserbecken angrenzen und von dem Wasserbecken aus mit Feuchtigkeit versorgt werden können. Das Wasserbecken kann auch so ausgebildet sein, dass das Wasserbecken einen Trinkwasservorrat für die Lebewesen in der Ansiedlungsvorrichtung bereithält.

Das Wasserbecken kann von den umgebenden Seitenwänden gehalten und mechanisch belastbar eingefasst sein. Auf diese Weise können auch vergleichsweise große Wassermengen in dem Innenraum der Ansiedlungsvorrichtung gespeichert sein.

Dadurch wird es ermöglicht, ein moorartiges Biotop innerhalb der Ansiedlungsvorrichtung zu erzeugen.

In Abhängigkeit von dem jeweiligen Verwendungszweck der Ansiedlungsvorrichtung kann vorgesehen sein, dass an der Oberseite der Ansiedlungsvorrichtung eine Dachplatte angeordnet ist. Die Dachplatte kann beispielsweise trichterförmig ausgebildet sein oder mehrere trichterförmige Sammelbereiche für Regenwasser aufweisen, um durch das von der Dachplatte aufgesammelte Regenwasser ein in dem Innenraum angeordnetes Wasserbecken zu befüllen.

Es kann auch vorgesehen sein, dass mit der Dachplatte eine mechanisch belastbare Abdeckung der Ansiedlungsvorrichtung bereitgestellt wird, sodass weitere Objekte auf der Oberseite der Ansiedlungsvorrichtung angeordnet werden können. Insbesondere kann mit einer geeignet ausgestaltete Dachplatte ein Aufeinanderstapeln von mehreren Ansiedlungsvorrichtungen begünstigt werden. Auf diese Weise kann mit mehreren zunächst getrennt hergestellten und angelieferten Ansiedlungsvorrichtungen ein großvolumiger Verbund von Ansiedlungsvorrichtungen errichtet werden, der die Ansiedlung von zahlreichen und vielen verschiedenen kleinen Lebewesen ermöglicht und begünstigt.

Einer Ausgestaltung des Erfindungsgedankens zufolge kann vorgesehen sein, dass an einer Außenseite der Abschirmungswand oder von gegebenenfalls mehreren Abschirmungswänden nach außen vorspringende Ansiedlungselemente angeordnet sind. Die Ansiedlungselemente können ebenso wie die Zugangsbeschränkungsdeckel entweder einstückig mit der betreffenden Abschirmungswand ausgebildet oder gesondert hergestellt und dauerhaft mit der Abschirmungswand verbunden sein. Es ist ebenfalls möglich, dass die nach außen vorspringenden Ansiedlungselemente nachträglich an der Abschirmungswand festgelegt werden können. Es kann sich um völlig unterschiedlich ausgestaltete Ansiedlungselemente handeln. Für Pflanzen eignen sich gitterförmige oder trogförmige Ansiedlungselemente, in denen oder an denen ein Pflanzenwachstum begünstigt wird. Für Tiere können vorspringende Lauf- und Kletterelemente für laufende, kriechende oder krabbelnde Tiere oder aber Überhänge geeignet sein, an denen Vögel an der Abschirmungswand ein Nest bauen können.

Weiterhin kann optional vorgesehen sein, dass an der Abschirmungswand gangartige oder plattenförmige Hohlräume ausgebildet sind, die mit einem Füllmaterial verfüllt sind. Auf diese Weise kann zusätzliches Füllmaterial in geschlossene oder verschließbare Hohlräume in der Abschirmungswand eingebracht werden. Die Abschirmungswand kann auch als eine Doppelwandstruktur ausgebildet sein. Optional kann in der Doppelwandstruktur eine vertikale Wabenstruktur ausgebildet sein. In dieser als Doppelwand ausgebildeten Abschirmungswand kann ein feines Pulver oder eine Schlemme eingebracht werden, wodurch eine Dichtheit der Abschirmungswand verbessert und deutlich erhöht werden kann. Es ist ebenfalls denkbar, in die Hohlräume oder in eine Doppelwandstruktur einen vorzugsweise biologisch abbaubaren Kunststoff einzuspritzen. Mit dem Füllmaterial kann zudem eine mechanische Stabilität und Belastbarkeit der Ansiedlungsvorrichtung verbessert und erhöht werden.

Die Ansiedlungsvorrichtung kann eine beliebige Außenkontur aufweisen. Die Ansiedlungsvorrichtung kann beispielsweise tonnenförmig ausgestaltet sein, wobei die Abschirmungswand und die Öffnungswand jeweils Bereiche der zylinderförmigen Außenwandfläche sind. Bei einer tonnenförmigen Ausgestaltung können bezogen auf das Raumvolumen der Ansiedlungsvorrichtung großflächige Öffnungswände ausgebildet werden, mit denen eine Ansiedlung von Tieren und Pflanzen begünstigt wird. In Abhängigkeit von der jeweiligen Herstellung der Ansiedlungsvorrichtung können insbesondere mit einem additiven Fertigungsverfahren nahezu beliebige Formgebungen und Volumina mit ganz unterschiedlichen geometrischen Grundformen realisiert werden. Die Ansiedlungsvorrichtung kann neben einer für viele Anwendungsfälle vorteilhaften quaderförmigen oder tonnenförmigen Ausgestaltung beispielsweise auch eine kegelförmige, eine kugelförmige oder eine prismenförmige Ausgestaltung denkbar ist.

Zweckmäßigerweise ist ein Raumbedarf einer Ansiedlungsvorrichtung so vorgegeben, dass die betreffende Ansiedlungsvorrichtung auf einem LKW oder in einem Überseecontainer transportiert werden kann. Im Hinblick auf eine möglichst einfache Handhabung der Ansiedlungsvorrichtung bei der Errichtung an dem gewünschten Aufstellungsort können auch deutlich kleinere Volumina von beispielsweise etwas mehr oder weniger als ein Kubikmeter vorteilhaft sein.

Für viele Anwendungsbereiche ist eine einfache und raumsparende Anordnung von mehreren Ansiedlungsvorrichtungen zweckmäßig, die miteinander verbunden und beispielsweise übereinander gestapelt oder nebeneinander aufgestellt werden können. Gemäß einer vorteilhaften Ausgestaltung des Erfindungsgedankens ist deshalb optional vorgesehen, dass die Ansiedlungsvorrichtung quaderförmig ausgebildet ist und zwei einander gegenüberliegend angeordnete Abschirmungswände sowie zwei einander gegenüberliegend angeordnete Öffnungswände aufweist. Die einzelnen Ansiedlungsvorrichtungen können dann raumsparend mit einander zugewandten Abschirmungswänden nebeneinander auf einem Untergrund oder auf einer darunter angeordneten Reihe von Ansiedlungsvorrichtungen aufgestellt werden und einen reihenförmigen oder mauerförmigen Verbund von Ansiedlungsvorrichtungen bilden, deren Öffnungswände jeweils frei zugänglich sind.

Im Hinblick auf eine möglichst hohe Stabilität und mechanische Belastbarkeit der Ansiedlungsvorrichtung kann vorgesehen sein, dass die Ansiedlungsvorrichtung zwei oder mehr Abschirmungswände und zwei oder mehr Öffnungswände aufweist, die in Umfangsrichtung abwechselnd aneinander angrenzend angeordnet und ausgebildet sind. Dabei können aneinander angrenzende Abschirmungswände und Öffnungswände über eine Seitenkante hinweg miteinander verbunden sein oder ineinander übergehen, sodass die aneinander angrenzenden Außenseitenbereiche einen Winkel zueinander aufweisen. Die aneinander angrenzenden Abschirmungswände und Öffnungswände können auch flächenbündig ineinander übergehen. Für viele Anwendungsfälle dürfte es zweckmäßig sein, dass die Ansiedlungsvorrichtung zwei einander gegenüberliegend angeordnete Abschirmungswände und zwei ebenfalle einander gegenüberliegend und jeweils zwischen zwei Abschirmungswänden angeordnete Öffnungswände aufweist.

Um zu ermöglichen, dass sich frische Luft und kleine Tiere zwischen unmittelbar nebeneinander aufgestellten Ansiedlungsvorrichtungen hindurch bewegen können ist optional vorgesehen, dass Kanten zwischen dem Boden, den Seitenwänden und dem Dach eine Fase aufweisen. Die einzelnen Fasen bilden bei zwei oder mehreren aneinander angrenzend angeordneten Ansiedlungsvorrichtungen zweckmäßigerweise eine Durchgangsöffnung mit einer lichten Weite, die mehr als 25% und vorzugsweise mehr als 50% einer Querschnittsfläche eines benachbart angeordneten Ansiedlungsgangs entspricht. Für viele Anwendungsfälle kann es vorteilhaft sein, wenn die Fasen aneinander angrenzend angeordneter Ansiedlungsvorrichtungen eine Querschnittsfläche aufweisen, die näherungsweise einer größten Querschnittsfläche einer Öffnung eines Ansiedlungsgangs entsprechen. Durch die Fasen wird ein Verbund von unmittelbar aneinander angrenzend errichteten Ansiedlungsvorrichtungen durchlässig für beispielsweise Luft oder kleine Tiere, ohne dass beispielsweise bei der Anordnung der einzelnen Ansiedlungsvorrichtungen auf einen Mindestabstand geachtet werden müsste. Zudem können benachbarte Ansiedlungsvorrichtungen flächig aneinander anliegend angeordnet oder mit gesonderten Verbindungsmitteln miteinander verbunden werden, um eine Verbundwirkung zu erzeugen, ohne dass dadurch dieser Verbund für Luft oder Tiere undurchlässig wird.

Einer besonders vorteilhaften Ausgestaltung des Erfindungsgedankens ist vorgesehen, dass die Ansiedlungsvorrichtung aus einem die Ansiedlung von Lebewesen begünstigenden Material hergestellt oder mit einem solchen Material beschichtet ist. Die Ansiedlungsvorrichtung kann beispielsweise überwiegend oder vollständig aus plattenförmigen Gangwänden zusammengefügt werden, die aus einem Keramikmaterial oder aus einem geschäumten Keramikmaterial hergestellt sind. Die Ansiedlungsvorrichtung kann auch aus einem Kunststoffmaterial, vorzugsweise aus einem geschäumten Kunststoffmaterial hergestellt sein, welches nachträglich gegebenenfalls mit einer geeigneten Beschichtung überzogen ist. Für viele Anwendungsfälle wird es als vorteilhaft erachtet, dass die Ansiedlungsvorrichtung vorzugsweise in Leichtbauweise ausgestaltet ist und aus einem Material mit einem möglichst geringen Eigengewicht und dennoch großer Formstabilität und mechanischer Belastbarkeit hergestellt ist. Es wird ebenfalls als vorteilhaft erachtet, wenn die Ansiedlungsvorrichtung aus einem porösen Material hergestellt ist oder eine offenporige Materialstruktur aufweist. Auf diese Weise kann eine durch die Poren und durch die Kapillarwirkung begünstigt eine vorteilhafte Wassertransportfähigkeit durch das Material selbst bewirkt werden, sodass die einzelnen Ansiedlungsgänge mit von außen eindringendem Wasser bzw. mit Feuchtigkeit versorgt werden können. Bei der Ausbildung eines Wasserbeckens in dem Innenraum der Ansiedlungsvorrichtung kann auch Wasser aus diesem Wasserbecken entnommen und durch die poröse Materialstruktur über die gesamte Ansiedlungsvorrichtung verteilt werden. Das poröse Material kann auch begünstigen, dass Sauerstoff in den Innenraum der Ansiedlungsvorrichtung gelangt und sich dort verteilen kann.

Vorzugsweise ist vorgesehen, dass mehrere Ansiedlungsgänge mindestens teilweise mit einem die Ansiedlung von Lebewesen begünstigenden Substrat befüllt sind. Bei dem Substrat kann es sich beispielsweise um Humus, Erde oder Sand bzw. um eine Mischung solcher Bodenbestandteile handeln. Das Substrat kann auch Steine aufweisen oder ausschließlich aus Steinen bestehen. Es kann auch vorgesehen sein, dass das Substrat geeignete Düngemittel oder ein Pflanzenwachstum fördernde Substanzen enthält. Das Substrat kann auch Pflanzenreste wie beispielsweise Rinde, Gräser oder Laub enthalten, um die Ansiedlung von Insekten, Vögeln oder kleinen Tieren wie etwa Siebenschläfern oder Eichhörnchen zu begünstigen. Durch eine künstliche Befüllung der Ansiedlungsgänge mit einem geeigneten Substrat können die betreffenden Ansiedlungsgänge sofort nach der Errichtung der Ansiedlungsvorrichtung Pflanzen und Tieren ein vorteilhaftes Lebensumfeld bieten und dadurch die Ansiedlung begünstigen. Es kann auch vorgesehen sein, dass die Ansiedlungsgänge zunächst leer zur Verfügung gestellt werden und von den sich dort ansiedelnden Lebewesen selbst mit einem Substrat befüllt bzw. mit einem geeigneten Nistmaterial befüllt werden.

Nachfolgend werden Ausführungsbeispiele des Erfindungsgedankens näher erläutert, die exemplarisch in den Zeichnungen dargestellt sind. Es zeigt:
Fig. 1 eine perspektivische Ansicht einer exemplarischen Ausgestaltung einer Ansiedlungsvorrichtung,
Fig. 2 eine Schnittansicht durch die in Fig. 1 gezeigte Ansiedlungsvorrichtung längs einer Schnittebene II-II in Fig. 1,
Fig. 3 eine perspektivische Ansicht der in Fig. 1 dargestellten Ansiedlungsvorrichtung mit mehreren Zugangsbeschränkungsdeckeln, die einzelne Öffnungen zu sich in einen Innenraum der Ansiedlungsvorrichtung erstreckenden Ansiedlungsgängen bedecken,
Fig. 4 eine perspektivische Ansicht von einer Öffnungswand mit mehreren Öffnungen sowie in auseinandergezogener Darstellung gezeigten mehreren Zugangsbeschränkungsdeckeln,
Fig. 5 eine perspektivische Ansicht einer als Abschirmungswand ausgebildeten Seitenwand der Ansiedlungsvorrichtung mit seitlich angeordneten Ansiedlungselementen sowie zwei in auseinandergezogener Darstellung separat dargestellte Ansiedlungselemente,
Fig. 6 eine exemplarische Anordnung mehrerer Ansiedlungsvorrichtungen, die nebeneinander und übereinander angeordnet eine Ansiedlungswand bilden,
Fig. 7 eine vergrößerte Teilansicht eines Bereichs VII in Fig. 6,
Fig. 8 eine vergrößerte Teilansicht eines Bereichs VIII in Fig. 6,
Fig. 9 eine perspektivische Ansicht einer Ansiedlungsvorrichtung mit mehreren von dem Innenraum heraus über eine Außenseite der Öffnungswand vorspringenden Gangwänden, die eine Wandabschirmung bildet,
Fig. 10 eine Schnittansicht durch eine Abschirmungswand und angrenzende Bereiche der in Fig. 9 gezeigten Ansiedlungsvorrichtung längs einer Linie X-X in Fig. 8,
Fig. 11 eine Schnittansicht durch die in Fig. 10 gezeigte Abschirmungswand längs einer Linie XI-XI in Fig. 9,
Fig. 12 eine perspektivische Ansicht einer Ansiedlungsvorrichtung mit einem mittig angeordneten Wasserbecken,
Fig. 13 eine weitere perspektivische Ansicht der in Fig. 12 gezeigten Ansiedlungsvorrichtung,
Fig. 14 eine perspektivische Ansicht einer exemplarisch dargestellten Ansiedlungsvorrichtung mit einer Dachplatte,
Fig. 15 eine perspektivische Ansicht einer vergleichbaren Ansiedlungsvorrichtung mit einer Trichterplatte,
Fig. 16 eine perspektivische Ansicht einer vergleichbaren Ansiedlungsvorrichtung ohne eine Dachplatte oder Trichterplatte,
Fig. 17 eine perspektivische Ansicht einer Ansiedlungsvorrichtung, deren Abmessungen an einen Überseecontainer angepasst sind,
Fig. 18 eine exemplarische Zusammenstellung verschiedener geometrischer Grundformen, die jeweils als Grundlage für eine Formgebung einer Ansiedlungsvorrichtung verwendet werden können,
Fig. 19 eine perspektivische Schnittansicht durch eine Ansiedlungsvorrichtung mit Ansiedlungsgängen, die nicht alle verschlossen sind und die über Gangwechselöffnungen den Übergang zwischen benachbarten Ansiedlungsgängen ermöglichen,
Fig. 20 eine perspektivische Schnittansicht der in Fig. 19 gezeigten Ansiedlungsvorrichtung mit einer oben angeordneten Dachplatte,
Fig. 21 eine Schnittansicht eines Teilbereichs der in Fig. 20 gezeigten Ansiedlungsvorrichtung um einen Randbereich der Dachplatte, und
Fig. 22 eine Schnittansicht durch eine abweichend ausgestaltet Ansiedlungsvorrichtung mit Ansiedlungsgängen.

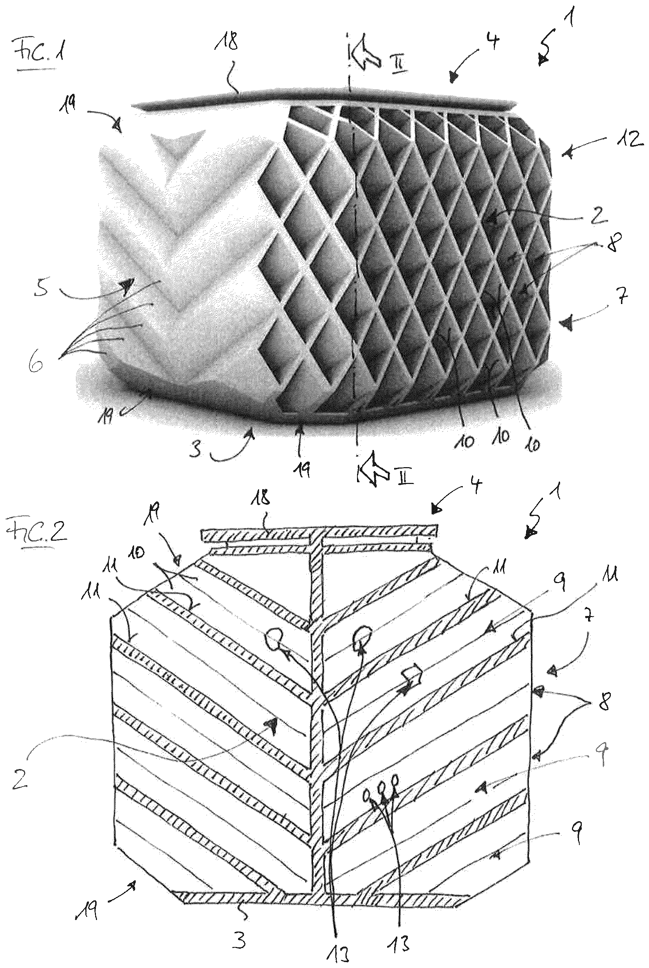

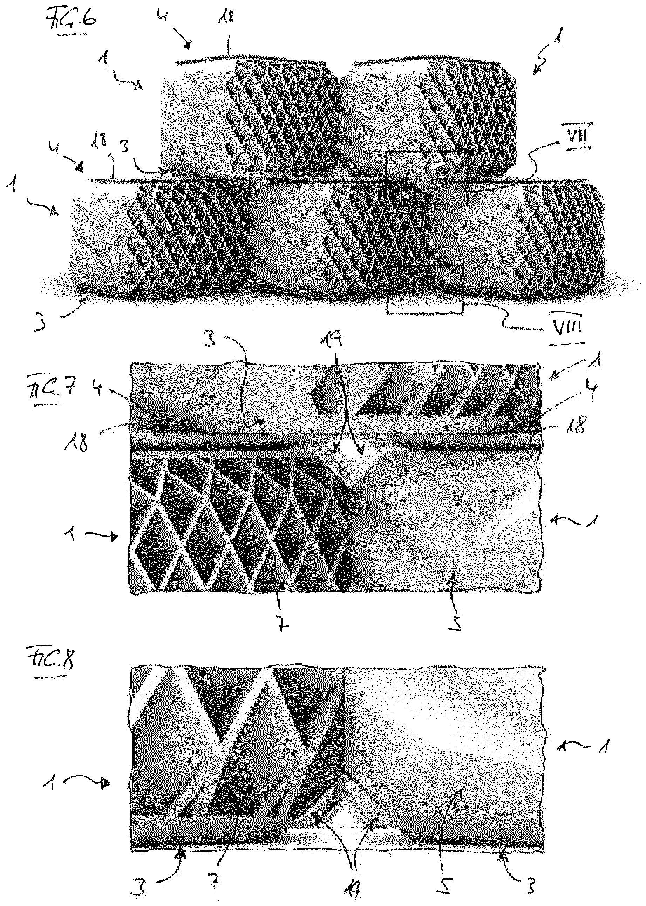

In den Fig. 1 bis 8 sind verschiedene Ansichten und Ausgestaltungen einer Ansiedlungsvorrichtung 1 dargestellt, die auf einem Untergrund errichtet werden kann und zur Unterstützung der Ansiedlung von Insekten, Vögeln und kleinen Säugetieren dient. Die Ansiedlungsvorrichtung 1 weist einen Innenraum 2 auf, der von einem auf dem Untergrund aufstellbaren Boden 3 und einem gegenüberliegenden Dach 4 sowie von vier sich jeweils von dem Boden 3 bis zu dem Dach 4 erstreckenden Seitenwänden umgeben ist. Zwei einander gegenüberliegende Seitenwände sind als Abschirmungswände 5 ausgestaltet, die durch Wandelemente 6 verschlossen sind. Zwei weitere, ebenfalls einander gegenüberliegende und auf beiden Seiten an eine Abschirmungswand 5 angrenzende Seitenwände bilden Öffnungswände 7, die jeweils eine Anzahl von Öffnungen 8 aufweisen, durch welche hindurch der Innenraum zugänglich ist.

In jeder Öffnungswand 7 ist hinter mehreren Öffnungen 8 jeweils ein Ansiedlungsgang 9 ausgebildet, der durch die zugeordnete Öffnung 8 zugänglich ist und der sich ausgehend von der betreffenden Öffnung 8 in den Innenraum hinein erstreckt. Jeder Ansiedlungsgang 9 ist an einem der Öffnung 8 entgegenliegenden Ende des Ansiedlungsgangs 9 verschlossen und durch Gangwände 10 von anderen Ansiedlungsgängen 9 getrennt. Alle Ansiedlungsgänge 9, die von einer Öffnungswand 7 aus zugänglich sind, weisen jeweils einen Gangboden 11 mit einem sich von der Öffnung 8 in den Innenraum hinein zu dem Boden 3 hin gerichteten Ganggefälle von etwa 30° relativ zu dem Boden 3 der Ansiedlungsvorrichtung 1 auf. Für viele Anwendungsfälle ist ein Ganggefälle zwischen 5° und 33°, vorzugsweise zwischen 20° und 30° vorteilhaft.

Jede Öffnungswand 7 weist eine große Anzahl von unmittelbar aneinander angrenzend angeordneten Öffnungen 8 auf, die lediglich durch dünne Gangwände 10 begrenzt und voneinander abgetrennt sind. Die Gangwände 10 bilden gleichzeitig auch die Begrenzungen für die einzelnen Ansiedlungsgänge 9 und insbesondere auch den Gangboden 11, der bei dem exemplarisch dargestellten Ausführungsbeispiel keine waagrechte Bodenplatte aufweist, sondern durch zwei V-förmig zueinander angeordnete Gangwände 10 gebildet wird. Die einzelnen Gangwände 10 sind jeweils plattenförmig ausgebildet und miteinander verbunden.

Die miteinander verbundenen Gangwände 10 bilden eine Stützstruktur 12 der Ansiedlungsvorrichtung 1, welche der Ansiedlungsvorrichtung 1 trotz eines vergleichsweise geringen Raumbedarfs und einer Leichtbauweise eine große Formstabilität und eine ausreichende mechanische Belastbarkeit verleihen. In dem exemplarisch dargestellten Ausführungsbeispiel weisen die Ansiedlungsgänge 9 jeweils eine einheitlich vorgegebene rautenförmige Querschnittsfläche auf. Es ist ebenfalls denkbar, dass die Ansiedlungsgänge 9 eine davon abweichende und beispielsweise eine wabenförmige, quaderförmige oder runde Querschnittsfläche aufweisen, oder dass benachbart angeordnete Ansiedlungsgänge 9 eine voneinander abweichende Querschnittsfläche aufweisen.

Mehrere nebeneinander angeordnete Ansiedlungsgänge 9 sind in dem Innenraum der Ansiedlungsvorrichtung 1 durch Gangwechselöffnungen 13 miteinander verbunden, die in Fig. 2 sichtbar sind. Diese Gangwechselöffnungen 13 sind so ausgebildet und angeordnet, dass kleine Lebewesen durch eine Gangwechselöffnung 13 von einem Ansiedlungsgang 9 in einen benachbarten Ansiedlungsgang 9 überwechseln können, ohne zuvor einen Ansiedlungsgang 9 durch die zugeordnete Öffnung 8 in der Öffnungswand 7 verlassen zu müssen, um dann den benachbarten Ansiedlungsgang 9 durch dessen Öffnung 8 betreten zu können. Zudem können sich Pflanzen und auch deren Wurzeln durch derartige Gangwechselöffnungen 13 über mehrere Ansiedlungsgänge 9 hinweg ausbreiten.

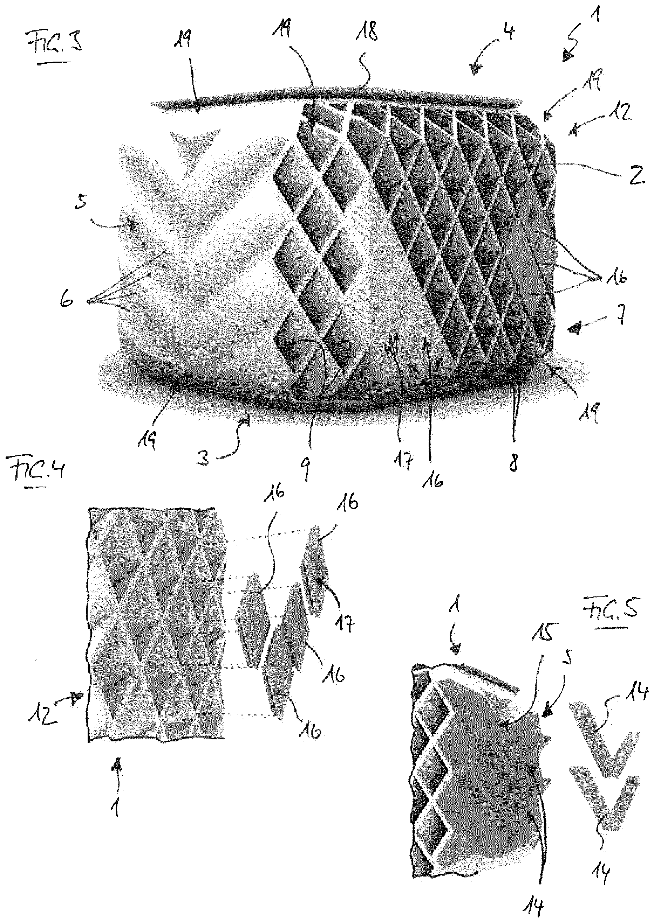

In Fig. 5 sind beispielhaft einige V-förmig ausgebildete und seitlich vorspringende Ansiedlungselemente 14 dargestellt, die an einer Außenseite 15 der Abschirmungswand 5 nach außen vorspringend angeordnet und an der Außenseite 15 festgelegt sind. Dadurch wird an der Außenseite 15 der vollständig geschlossenen Abschirmungswand 5 zusätzlich eine Ansiedlung von Vögeln und kleinen Tieren begünstigt.

Die Öffnung 8 eines jeden Ansiedlungsgangs 9 kann durch einen in den Fig. 3 und 4 dargestellten Zugangsbeschränkungsdeckel 16 abgedeckt sein. Ein Zugangsbeschränkungsdeckel 16 kann die Öffnung 8 eines Ansiedlungsgangs 9 vollständig verschließen, sodass der betreffende Ansiedlungsgang 9 ausschließlich über Gangwechselöffnungen 13 in dem Innenraum der Ansiedlungsvorrichtung 1 zugänglich ist. Ein Zugangsbeschränkungsdeckel 16 kann auch eine oder mehrere Zugangsöffnungen 17 aufweisen, durch welche hindurch ein Lebewesen sich in den Ansiedlungsgang 9 hinein oder aus dem Ansiedlungsgang 9 heraus bewegen kann. Die Abmessungen der Zugangsöffnungen 17 in einem Zugangsbeschränkungsdeckel 16 können an unterschiedliche Tierarten angepasst sein, sodass mit Hilfe der Zugangsbeschränkungsdeckel 16 für einzelne Ansiedlungsgänge 9 jeweils hinsichtlich der Größe vorgegebene Tierarten zugeordnet sein können, die sich überwiegend dort und seltener in anderen Ansiedlungsgängen 9 ansiedeln werden.

Die in den Fig. 1 bis 8 exemplarisch dargestellte Ansiedlungsvorrichtung 1 ist quaderförmig ausgebildet und weist an ihrem Dach 4 eine im Wesentlichen ebenflächig ausgebildete Dachplatte 18 auf. Entlang von Kanten, die seitlich längs des Bodens 3, längs der Seitenwände und längs des Dachs 4 verlaufen, ist jeweils eine Fase 19 ausgebildet. Durch die quaderförmige Kontur der Ansiedlungsvorrichtung 1 können mehrere Ansiedlungsvorrichtungen 1 in einfacher Weise nebeneinander und übereinander angeordnet werden, um eine in Fig. 5 exemplarisch dargestellte Ansiedlungswand 20 zu bilden, die aus einem Verbund von mehreren Ansiedlungsvorrichtungen 1 besteht. Auf der ebenflächig ausgebildeten und waagrecht ausgerichteten Dachplatte 18 von Ansiedlungsvorrichtungen 1, die nebeneinander auf dem Untergrund aufgestellt sind, kann eine zweite Reihe von weiteren Ansiedlungsvorrichtungen 1 angeordnet werden. Bei Bedarf könnten auch weitere Reihen von weiteren Ansiedlungsvorrichtungen 1 vorgesehen sein und eine noch höhere Ansiedlungswand 20 bilden. Durch die Fasen 19 werden zwischen benachbart angeordneten Ansiedlungsvorrichtungen 1 jeweils Durchgangsöffnungen 21 gebildet, durch welche Luft hindurchströmen kann und Tiere durch die Ansiedlungswand 20 hindurch sich bewegen können.

Jede Ansiedlungsvorrichtung 1 ist aus einem die Ansiedlung von Lebewesen begünstigenden Material hergestellt oder mit einem solchen Material beschichtet ist. Mehrere Ansiedlungsgänge 9 sind mindestens teilweise mit einem die Ansiedlung von Lebewesen begünstigenden Substrat befüllt.

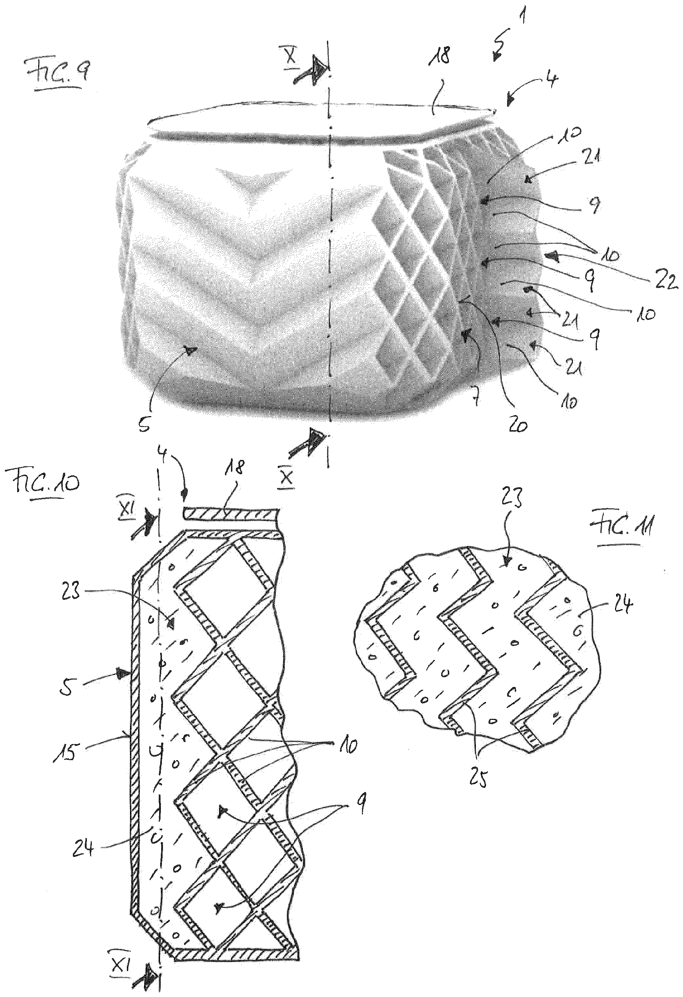

In Fig. 9 ist exemplarisch eine ebenfalls quaderförmig ausgebildete Ansiedlungsvorrichtung 1 dargestellt. In einer Öffnungswand 7 mit einer ansonsten ebenflächig ausgestalteten Außenseite 20 sind mehrere aneinander angrenzend angeordnete Gangwände 10 mehrerer ebenfalls aneinander angrenzend angeordneten Ansiedlungsgänge 9 von dem Innenraum heraus über die Außenseite 20 der Öffnungswand 7 vorspringend ausgebildet. Die vorspringenden Bereiche 21 der Gangwände 10 bilden eine Wandabschirmung 22 zwischen Öffnungen 8 auf gegenüberliegenden Seiten der Wandabschirmung 22. Zudem kann die Wandabschirmung 22 eine Verschattung der angrenzenden Bereiche der Öffnungswand 7 und damit für die in den angrenzenden Bereichen angeordneten Öffnungen 8 mit den dahinter ausgebildeten Ansiedlungsgängen 9 bewirken.

In den Fig. 10 und 11 sind zwei Schnittansichten einer Abschirmungswand 5 mit angrenzenden Bereichen der in Fig. 9 gezeigten Ansiedlungsvorrichtung 1 dargestellt. Die Abschirmungswand 5 ist doppelwandig ausgebildet und weist einen plattenartig ausgebildeten Hohlraum 23 zwischen der Außenseite 15 und den benachbart angeordneten Ansiedlungsgängen 9 auf. Dieser Hohlraum 23 ist mit einem Füllmaterial 24 aufgefüllt. In dem Hohlraum 23 können wellenförmige oder wabenförmige Stützstrukturen 25 ausgebildet sein, wie es in Fig. 11 beispielhaft dargestellt ist.

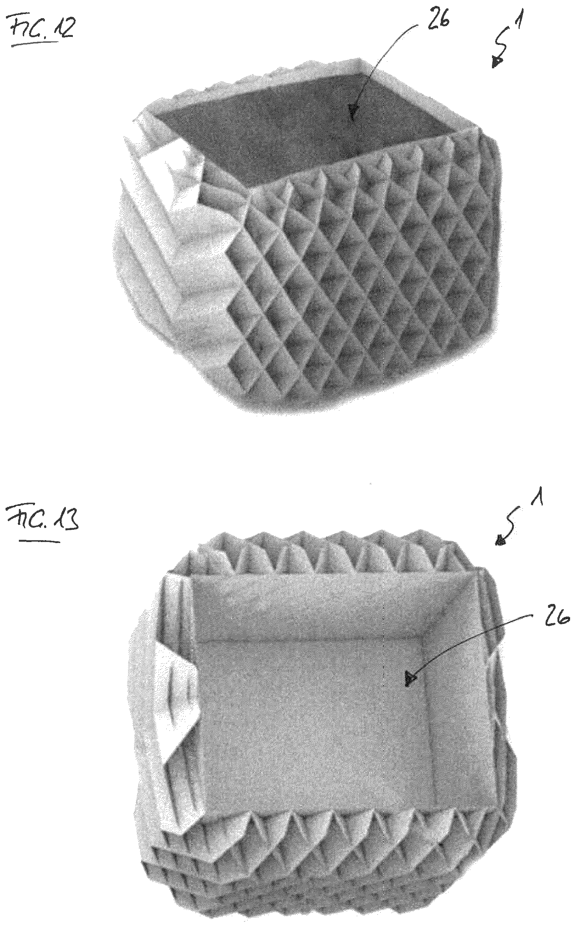

In den Fig. 12 und 13 sind zwei perspektivische Ansichten einer Ansiedlungsvorrichtung 1 mit einem mittig angeordneten Wasserbecken 26 dargestellt. Das Wasserbecken 26 kann künstlich mit Wasser befüllt werden oder auf die Ansiedlungsvorrichtung 1 herabregnendes Regenwasser aufsammeln und speichern. Das Wasserbecken 26 kann eine beliebige Formgebung aufweisen und wenig oder viel Volumen des Innenraums der Ansiedlungsvorrichtung 1 umfassen. Bei dem exemplarisch dargestellten Ausführungsbeispiel weist das Wasserbecken 26 ein vergleichsweise großes Volumen auf, sodass die Ansiedlungsvorrichtung 1 wie ein künstlicher Teich oder ein künstliches Moor verwendet werden kann. Es ist ebenfalls möglich, dass das Wasserbecken 26 durch Trennwände in mehrere Beckenbereiche unterteilt ist. Es können auch mehrere kleinere Wasserbecken 26 in dem Innenraum 2 der Ansiedlungsvorrichtung 1 ausgebildet und angeordnet sein.

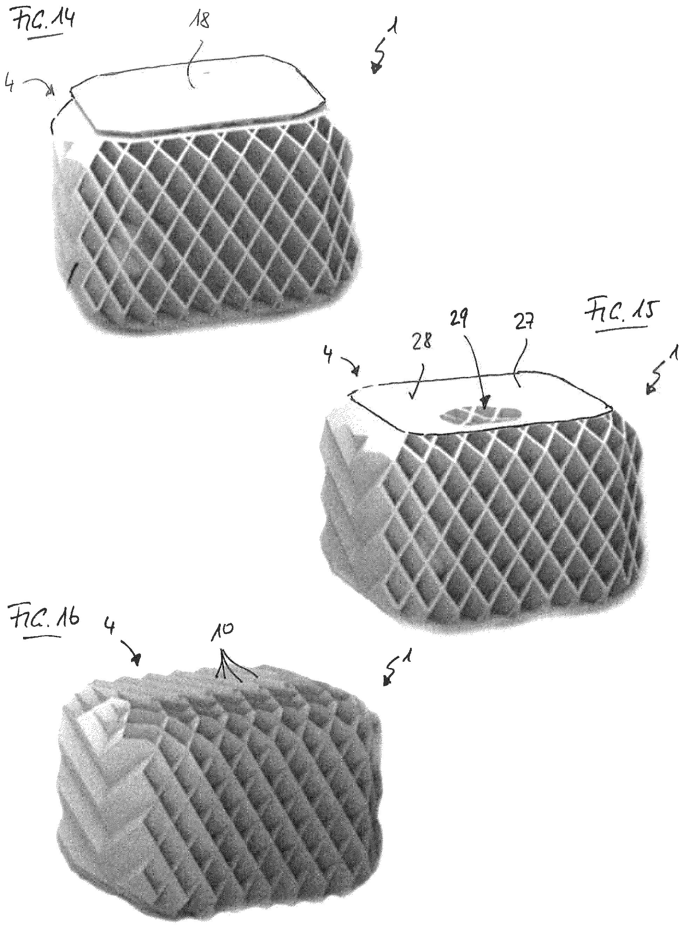

In den Fig. 14 bis 16 sind exemplarisch mehrere ansonsten vergleichbare Ansiedlungsvorrichtungen 1 dargestellt, deren Oberseite 4 unterschiedlich ausgestaltet ist. Bei dem in Fig. 14 dargestellten Ausführungsbeispiel ist an der Oberseite 4 der Ansiedlungsvorrichtung eine im Wesentlichen ebenflächige Dachplatte 18 angeordnet. Auf dieser Dachplatte 18 können in einfacher Weise andere Objekte oder weitere Ansiedlungsvorrichtungen 1 angeordnet werden.

Bei dem in Fig. 15 dargestellten Ausführungsbeispiel ist an der Oberseite 4 eine Trichterplatte 27 angeordnet, deren Außenseite 28 ein Gefälle zu einer mittig angeordneten Trichterabflussöffnung 29 aufweist. Mit einer derartigen Trichterplatte 27 kann darauf auftreffendes Regenwasser effizient gesammelt und dem Innenraum der Ansiedlungsvorrichtung 1 bzw. einem darin angeordneten Wasserbecken 26 zugeführt werden.

Bei dem in Fig. 16 dargestellten Ausführungsbeispiel wird die Oberseite 4 durch die Gangwände 10 der Ansiedlungsgänge 9 gebildet.

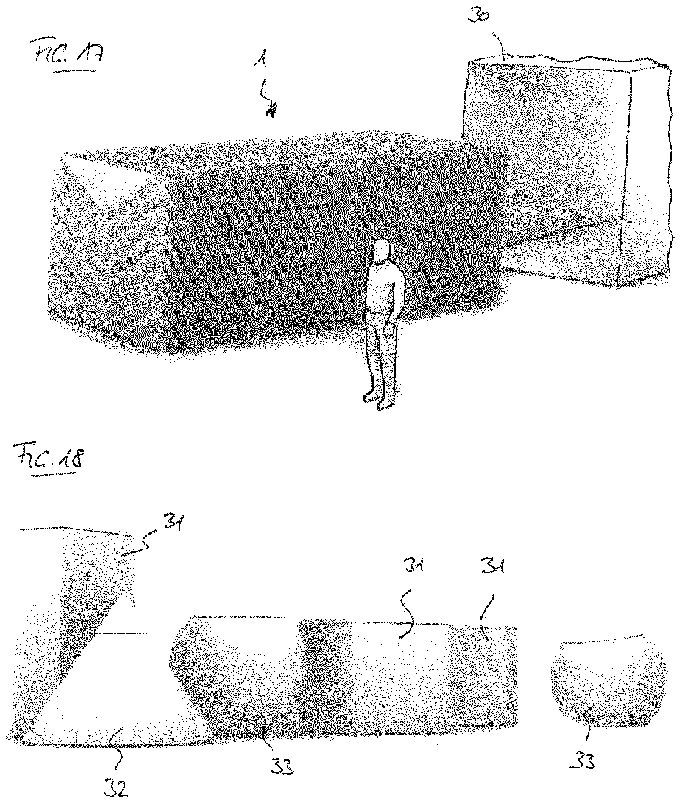

In Fig. 17 ist beispielhaft eine Ansiedlungsvorrichtung 1 dargestellt, deren Formgebung und Abmessungen an ein Nutzvolumen eines Überseecontainers 30 angepasst ist.

In Fig. 18 sind zur Veranschaulichung verschiedene geometrische Grundformen wie beispielsweise Quader 31, Kegel 32 oder Kugeln 33 dargestellt, die jeweils als Grundlage für eine Formgebung einer Ansiedlungsvorrichtung 1 verwendet werden können.

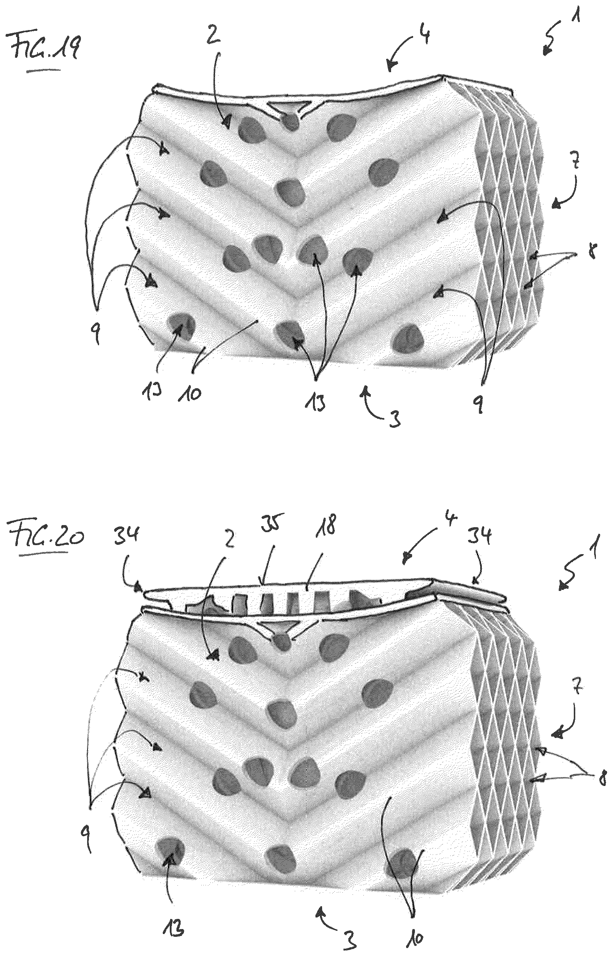

In Fig. 19 ist eine perspektivische und geschnittene Ansicht eines wiederum abweichend ausgestalteten Ausführungsbeispiels für eine Ansiedlungsvorrichtung 1 dargestellt. Alle einzelnen Ansiedlungsgänge 9 weisen in den Gangwänden 10 jeweils Gangwechselöffnungen 13 zu benachbart angeordneten Ansiedlungsgängen 9 auf. Auf diese Weise sind die mehreren Ansiedlungsgänge 9 untereinander verbunden. Jeder einzelne Ansiedlungsgang 9 ist über den Innenraum 2 der Ansiedlungsvorrichtung 1 aus zugänglich. Ein Lebewesen kann sich in einem Ansiedlungsgang 9 aufhalten oder dort ansiedeln, ohne dass das Lebewesen notwendigerweise diesen Ansiedlungsgang 9 nur über die zugeordnete Öffnung 8 in der Öffnungswand 8 erreichen könnte.

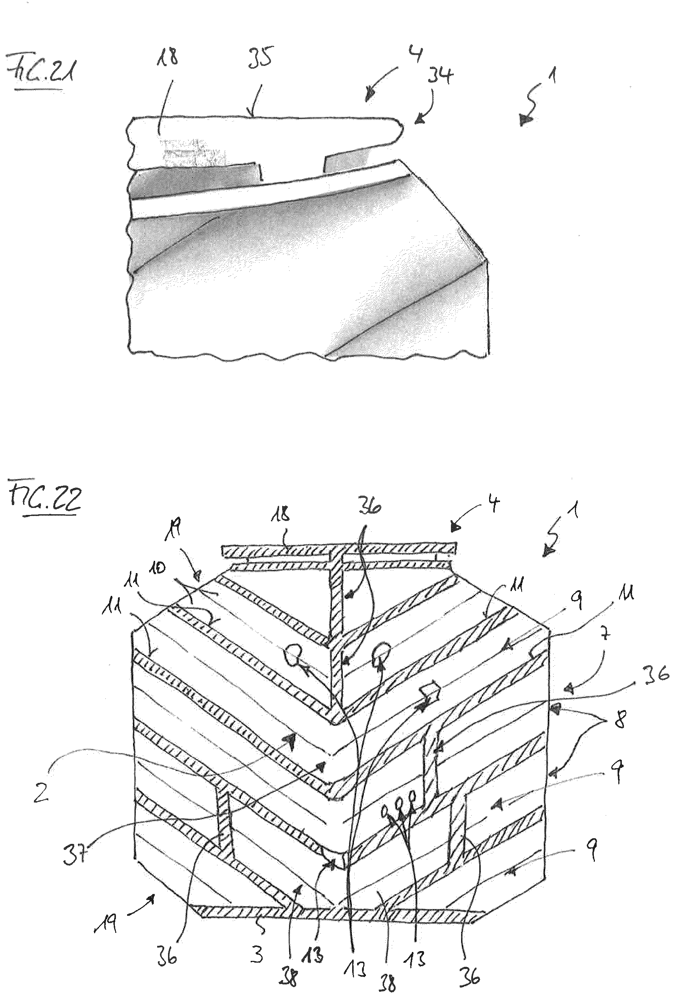

In Fig. 20 ist eine weitere Ansicht der in Fig. 19 gezeigten Ansiedlungsvorrichtung 1 dargestellt, wobei auf dem Dach 4 der Ansiedlungsvorrichtung 1 eine in Fig. 21 im Detail und vergrößert dargestellte Dachplatte 18 mit einer sich entlang des gesamten Umfangs erstreckenden abgerundeten Randkante 34 angeordnet ist. Die Dachplatte 18 ist bei dem exemplarisch dargestellten Ausführungsbeispiel lose auf das Dach 4 aufgelegt, wobei formschlüssige Eingriffe verhindern, dass die Dachplatte 18 verrutscht oder sich unbeabsichtigt von dem Dach 4 ablöst. Eine Oberseite 35 der Dachplatte 18 weist eine geringe konvexe und nach oben vorspringende Wölbung auf. Auf die Oberseite 35 der Dachplatte 18 auftreffendes Regenwasser oder kondensierende Feuchtigkeit läuft dann zum Rand der Dachplatte 18 und um die abgerundete Randkante 34 zu einem weiter innenliegenden Bereich der Dachplatte 18 und kann von dort in den Innenraum 2 der Ansiedlungsvorrichtung 1 geführt werden. Auf diese Weise kann Wasser gesammelt und dem Innenraum 2 der Ansiedlungsvorrichtung 1 zugeführt werden, ohne dass beispielsweise herabfallendes Laub diese Wasserzuführung verstopfen könnte.

In Fig. 22 ist eine mit Fig. 2 vergleichbare Schnittansicht eines weiteren Ausführungsbeispiels für die Ansiedlungsvorrichtung 1 dargestellt. Im Unterschied zu der in Fig. 2 dargestellten Ansiedlungsvorrichtung 1 sind nicht alle Ansiedlungsgänge 9 in einem mittleren Bereich durch eine Abschlusswand 36 verschlossen, welche die von einander gegenüberliegenden Öffnungswänden 7 jeweils zu einem mittigen Bereich des Innenraums 2 führenden Ansiedlungsgänge 9 in zwei etwa gleich große Ansiedlungsgänge 9 aufteilt. Es ist ebenfalls möglich, dass ein durchgehender Ansiedlungsgang 9 in einem mittigen Bereich 37 offen ist bzw. einen Durchgang aufweist. Dieser durchgehende Ansiedlungsgang 9 weist lediglich in dem mittigen Bereich 37 eine Richtungsänderung auf, sodass die einander gegenüberliegenden Öffnungen 8 nicht durch eine innerhalb des Ansiedlungsgangs 9 verlaufende Gerade miteinander verbunden werden könnten und dem zufolge auch keine Sichtverbindung der beiden Öffnungen 8 besteht.

Bei anderen Ansiedlungsgängen 9 können außermittig jeweils eine Abschlusswand 36 angeordnet sein und den betreffenden Ansiedlungsgang 9 auf eine vergleichsweise geringe Länge begrenzen.

Es ist ebenfalls möglich, dass durch mehrere Abschlusswände 36 ein innenliegender Abschnitt 38 eines Ansiedlungsgangs 9 vollständig von einer zugeordneten Öffnungswand 7 abgeschnitten wird und ausschließlich über eine Gangwechselöffnung 13 zugänglich ist.

## Patentansprüche

1. Ansiedlungsvorrichtung (1) zur Unterstützung der Ansiedlung von Insekten, Vögeln und kleinen Säugetieren sowie Amphibien, Reptilien, Pflanzen und Pilzen oder anderen kleinen Lebewesen, mit einem Innenraum, der von mindestens zwei Seitenwänden umgeben ist, die sich von einem auf einem Untergrund aufstellbaren Boden (3) bis zu einer dem Boden (3) gegenüberliegenden Oberseite (4) der Ansiedlungsvorrichtung (1) erstrecken, wobei mindestens eine Seitenwand eine Abschirmungswand (5) bildet, die durch Wandelemente (6) verschlossen ist, und mindestens eine an die Abschirmungswand (5) angrenzende Seitenwand eine Öffnungswand (7) bildet, die jeweils eine Anzahl von Öffnungen (8) aufweist, durch welche hindurch der Innenraum zugänglich ist, **dadurch gekennzeichnet, dass** in der Öffnungswand (7) hinter mehreren Öffnungen (8) jeweils ein Ansiedlungsgang (9) ausgebildet und durch die zugeordnete Öffnung (8) zugänglich ist, der sich von der betreffenden Öffnung (8) in den Innenraum hinein erstreckt, und dass jeder Ansiedlungsgang (9) durch Gangwände (10) von anderen Ansiedlungsgängen (9) getrennt ist.

2. Ansiedlungsvorrichtung (1) nach Anspruch 1, **dadurch gekennzeichnet, dass** mehrere oder alle Ansiedlungsgänge (9) jeweils in einem Abstand von der zugeordneten Öffnung (8) verschlossen sind.

3. Ansiedlungsvorrichtung (1) nach Anspruch 1 oder Anspruch 2, **dadurch gekennzeichnet, dass** mehrere Ansiedlungsgänge (9), die von der Öffnungswand (7) aus zugänglich sind, jeweils einen Gangboden (10) mit einem sich von der Öffnung (8) in den Innenraum hinein zu dem Boden (3) hin gerichteten Ganggefälle zwischen 5° und 33°, vorzugsweise zwischen 20° und 30° relativ zu dem Boden (3) der Ansiedlungsvorrichtung (1) aufweisen.

4. Ansiedlungsvorrichtung (1) nach einem der voranstehenden Ansprüche, **dadurch gekennzeichnet, dass** die Öffnungswand (7) durch aneinander angrenzende Öffnungen (8) gebildet wird, die jeweils durch Gangwände (10) begrenzt sind, welche die sich von den Öffnungen (8) in den Innenraum erstreckenden Ansiedlungsgänge (9) begrenzen.

5. Ansiedlungsvorrichtung (1) nach einem der voranstehenden Ansprüche, **dadurch gekennzeichnet, dass** mindestens mehrere Ansiedlungsgänge (9) über mindestens einen Abschnitt des jeweiligen Ansiedlungsgangs (9) eine rautenförmige oder wabenförmige Querschnittsfläche aufweisen.

6. Ansiedlungsvorrichtung (1) nach einem der voranstehenden Ansprüche, **dadurch gekennzeichnet, dass** mehrere nebeneinander angeordnete Ansiedlungsgänge (9) durch Gangwechselöffnungen (13) miteinander verbunden sind.

7. Ansiedlungsvorrichtung (1) nach einem der voranstehenden Ansprüche, **dadurch gekennzeichnet, dass** die Öffnung (8) eines Ansiedlungsgangs (9) durch einen Zugangsbeschränkungsdeckel (16) abgedeckt ist, der eine oder mehrere Zugangsöffnungen (17) aufweist, durch welche hindurch ein Lebewesen sich in den Ansiedlungsgang (9) hinein oder aus dem Ansiedlungsgang (9) heraus bewegen kann.

8. Ansiedlungsvorrichtung (1) nach einem der voranstehenden Ansprüche, **dadurch gekennzeichnet, dass** mindestens eine Gangwand (10) eines Ansiedlungsgangs (9) von dem Innenraum heraus über eine Außenseite (20) der Öffnungswand (7) vorspringend ausgebildet ist, um eine Wandabschirmung (22) zwischen Öffnungen (8) auf gegenüberliegenden Seiten der vorspringenden Gangwand (10) zu bilden.

9. Ansiedlungsvorrichtung (1) nach einem der voranstehenden Ansprüche, **dadurch gekennzeichnet, dass** in dem Innenraum ein mit Wasser befüllbares Wasserbecken ausgebildet ist.

10. Ansiedlungsvorrichtung (1) nach einem der voranstehenden Ansprüche, **dadurch gekennzeichnet, dass** an der Oberseite (4) der Ansiedlungsvorrichtung (1) eine Dachplatte (18) angeordnet ist.

11. Ansiedlungsvorrichtung (1) nach einem der voranstehenden Ansprüche, **dadurch gekennzeichnet, dass** an einer Außenseite (15) der Abschirmungswand (5) nach außen vorspringende Ansiedlungselemente (14) angeordnet sind.

12. Ansiedlungsvorrichtung (1) nach einem der voranstehenden Ansprüche, **dadurch gekennzeichnet, dass** an der Abschirmungswand (5) gangartige oder plattenförmige Hohlräume ausgebildet sind, die mit einem Füllmaterial verfüllt sind.

13. Ansiedlungsvorrichtung (1) nach einem der voranstehenden Ansprüche, **dadurch gekennzeichnet, dass** die Gangwände (10) der Ansiedlungsgänge (9) zumindest bereichsweise plattenförmig ausgebildet sind und miteinander verbunden sind, um eine Stützstruktur (12) der Ansiedlungsvorrichtung (1) zu bilden, auf welcher bei einer bestimmungsgemäßen Nutzung der Ansiedlungsvorrichtung (1) eine weitere Ansiedlungsvorrichtung (1) abgestützt und getragen werden kann.

14. Ansiedlungsvorrichtung (1) nach einem der voranstehenden Ansprüche, **dadurch gekennzeichnet, dass** die Ansiedlungsvorrichtung (1) quaderförmig ausgebildet ist und zwei einander gegenüberliegend angeordnete Abschirmungswände (5) sowie zwei einander gegenüberliegend angeordnete Öffnungswände (7) aufweist.

15. Ansiedlungsvorrichtung (1) nach einem der voranstehenden Ansprüche, **dadurch gekennzeichnet, dass** Kanten zwischen dem Boden (3) und den Seitenwänden, zwischen den Seitenwänden und zwischen den Seitenwänden und der Oberseite (4) eine Fase (19) aufweisen.

16. Ansiedlungsvorrichtung (1) nach einem der voranstehenden Ansprüche, **dadurch gekennzeichnet, dass** die Ansiedlungsvorrichtung (1) aus einem die Ansiedlung von Lebewesen begünstigenden Material hergestellt oder mit einem solchen Material beschichtet ist.

17. Ansiedlungsvorrichtung (1) nach einem der voranstehenden Ansprüche, **dadurch gekennzeichnet, dass** mehrere Ansiedlungsgänge (9) mindestens teilweise mit einem die Ansiedlung von Lebewesen begünstigenden Substrat befüllt sind.
